# EUROPEAN PATENT SPECIFICATION

(11) **EP 1 803 441 B1**
(45) Date of publication and mention of the grant of the patent: **29.04.2009**
(21) Application number: 05258082.6
(22) Date of filing: 28.12.2005
(51) Int. Cl.: A61K 9/14, A61K 9/20, A61K 31/216

(54) **Pharmaceutical formulations of fenofibrate having improved bioavailability**
Pharmazeutische Formulierungen mit Fenofibrat mit verbesserter Bioverfügbarkeit
Formulation pharmaceutique comprenant du fénofibrate ayant une biodisponibilité ameliorée

(43) Date of publication of application: 04.07.2007
(73) Proprietor: Teva Pharmaceutical Industries Ltd, 49131 Petah Tiqva (IL)
(72) Inventor: Lerner, Itzhak E., Petach Tikva 49404 (IL); Rosenberger, Vered, Modiin, 71700 (IL); Flashner-Barak, Moshe, Petach Tikva 49313 (IL); Drabkin, Anna, Tzur Hadassa. 99875,P.O.B 10221 (IL); Moldavski, Naomi, D.N HaNegev, 85492 (IL)
(74) Representative: Gallagher, Kirk James

(56) References cited:
- WO-A-20/05034920
- US-A- 4 895 726
- US-A1- 2001 006 655
- US-A1- 2002 056 206
- US-A1- 2003 224 059
- US-B1- 6 277 405

## Description

### FIELD OF THE INVENTION

The present invention relates to pharmaceutical compositions that include fenofibrate, a polyethylene glycol, and a polyethylene-polypropylene glycol, wherein the composition is made by sublimation of a sublimable carrier from a solid solution containing fenofibrate, a polyethylene glycol, a polyethylene-polypropylene glycol, and a sublimation carrier like menthol.

### BACKGROUND OF THE INVENTION

Fenofibrate, (2-[4-(4-chlorobenzoyl) phenoxy]-2-methyl-propanoic acid, 1-methylethyl ester) is one of the fibrate class of drug. It is available as both capsules and tablets. Fenofibrate is apparently a prodrug. The active moiety is reportedly the metabolite fenofibric acid which is reported to be produced in the body by the action of esterases. When fenofibrate is administered, apparently no intact fenofibrate is found in the plasma (Physician's Desk Reference 58th ed. 2004 pages 522 - 525 (PDR)).

Fenofibrate has very poor solubility in water. That is, it is a poorly water soluble drug. Despite its poor solubility in water, it is reported to be absorbed to a therapeutically acceptable degree when dosed in the "fed state" but less so when dosed in the "fasted state". The true "bioavailability" of the metabolite fenofibric acid is uncertain because much of it is understood to be metabolized to the glucuronide in both presystemic and first pass sites.

The absolute bioavailability of fenofibrate cannot supposedly be determined because it is insoluble in media suitable for intravenous injection. Following oral administration in healthy volunteers, approximately 60% of a single dose of radiolabelled fenofibrate appeared in urine, primarily as fenofibric acid and its glucuronide conjugate, and 25% was excreted in the feces. (PDR) The absorption of fenofibrate is understood to be increased when administered with food. The extent of absorption from orally administered tablets is increased by approximately 35% when tablets are taken with food (PDR, Martindale 33rd ed. Page 889).

Attempts have been made to improve the formulation of fenofibrate, especially as regards the bioavailability of fenofibrate. United States Patents Nos. 4,895,726 and 5,880,148 disclose co-micronizing the fenofibrate with surface active agents. United States Patent Nos. 6,074,670 , 6,277,405 disclose micronized fenofibrate coated onto hydrosoluble carriers with optional surface active agents. United States Patent No. 6,814,977 discloses fenofibrate dissolved in a medium chain glycerol ester of fatty acid. United States Patent No. 6,719,999 discloses fenofibrate dissolved in glycerin, propylene glycol, or dimethylisosorbide and US Patent No. 5,827,536 discloses fenofibrate dissolved in diethyleneglycol monoethyl ether.

Several patents disclose specific formulations of micronized fenofibrate with specific polymeric or surface active agent additives and other patents describe emulsions and suspensions of fenofibrate. For example, US Patent Application Publication No. 20040087656 discloses fenofibrate of particle size less than 2000 nm claimed to have an improved bioavailability. US Patent Application Publication No. 20030224059 discloses microparticles of active pharmaceutical ingredients, drug delivery vehicles comprising same, and methods for making them.

Micronization of the fenofibrate and combinations of micronized fenofibrate with surface active agents have moderately raised the bioavailability of fenofibrate allowing the agency-approved amount of drug dosed to be reduced from 100 mg per dose to 67 mg per dose and then subsequently to 54 mg per dose, whilst maintaining bioavailability in the fed state. Nanoparticle formulations of the drug have further allowed the reduction of the dose to 48 mg per dose with the bioavailability of the "fasted state" being reported as similar to the fed state. There is still room for much improvement because it is postulated that the true bioavailability of fenofibrate is still relatively low.

### SUMMARY OF THE INVENTION

In one aspect, the present invention relates to a pharmaceutical composition comprising non-mechanically micronized microparticles of fenofibrate, especially sublimation micronized microparticles of fenofibrate using menthol as a sublimable carrier; polyethylene glycol, especially polyethylene glycol 6000; and a polyethylene-polypropylene glycol, especially poloxamer 407. The pharmaceutical composition can further include a pharmaceutical disintegrant selected from the group consisting of crospovidone, a carboxymethyl cellulose, especially crosslinked carboxymethylcellulose sodium (croscarmellose sodium), the bicarbonate or carbonate salts; especially alkali metal bicarbonates or carbonates like sodium bicarbonate, sodium carbonate, potassium bicarbonate, potassium carbonate, calcium carbonate, magnesium carbonate, especially sodium bicarbonate; the organic carboxylic acids, especially citric acid, tanic acid, ascorbic acid, benzoic acid, fumaric acid, lactic acid, malic acid, sorbic acid, and tartaric acid, especially citric acid and tartaric acid; and combinations of any of the foregoing.

In another aspect, the present invention relates to a solid oral dosage form including a pharmaceutical composition that includes about 15% to about 25% by weight of non-mechanically micronized microparticles of fenofibrate, especially sublimation micronized fenofibrate; about 7% to about 13% by weight poloxamer 407; about 7% to about 13% polyethylene glycol 6000; about 15% by weight microcrystalline cellulose; about 18% crospovidone by weight; about 12% sodium bicarbonate by weight; and about 12% by weight of either citric acid or tartaric acid.

In yet a further aspect, the present invention relates to a solid oral dosage form including a pharmaceutical composition that includes about 15% to about 25% by weight of non-mechanically micronized microparticles of fenofibrate, especially sublimation micronized fenofibrate; about 7% to about 13% by weight poloxamer 407; about 7% to about 13% polyethylene glycol 6000; about 15% by weight microcrystalline cellulose; about 18% crospovidone by weight; about 12% sodium bicarbonate by weight; and about 12% by weight of either citric acid or tartaric acid; wherein the dosage form has a time-dependent *in vitro* fenofibrate release profile such that at least about 51 % by weight, especially about 51 % to about 81% of the fenofibrate is released in about 10 minutes, at least about 73%, especially about 73% to about 93%, by weight of the fenofibrate is released in about 15 minutes, and at least about 85% by weight, especially about 85% by weight to essentially all of the fenofibrate is released in about 30 minutes.

In another aspect, the present invention relates to a solid oral dosage form, especially a compressed tablet, comprising a pharmaceutical composition that includes about 145 mg of sublimation micronized fenofibrate wherein in human *in vivo* pharmacokinetic studies in which the dosage form is administered in the fasted state, the area under the 48-hour AUC curve (AUC₄₈) is about 121367 h*ng/g to about 287539 h*ng/g; the area under the AUC curve extrapolated to infinite time (AUC_{∞}) is about 134750 h*ng/g to about 345390 h*ng/g; and the maximum plasma concentration (Cₘₐₓ) is about 6357 ng/g to about 14627 ng/g. Typically, such solid oral dosage form will exhibit an average AUC₄₈ of about 175335 h*ng/g, an average AUC_{∞} of about 213652 h*ng/g, and an average Cₘₐₓ of about 10570 ng/g.

In still yet another aspect, the present invention relates to a solid oral dosage form, especially a compressed tablet, that includes a pharmaceutical composition having about 145 mg of sublimation micronized fenofibrate wherein, in human *in vivo* pharmacokinetic studies in which the dosage form is administered in the fed state, the area under the 48-hour AUC curve (AUC₄₈) is about 91601 h*ng/g to about 217512 h*ng/g; the area under the AUC curve extrapolated to infinite time (AUC_{∞}) is about 97182 h*ng/g to about 308070 h*ng/g, and further wherein the average AUC₄₈ is about 150511 h*ng/g and the average AUC∞ is about 185149 h*ng/g. The dosage form can include a disintegrant.

In yet a further aspect, the present invention relates to a pharmaceutical composition having a plurality of pharmaceutical carrier particles, especially particles of microcrystalline cellulose, having deposited thereon a combination of fenofibrate; especially about 15% to about 25% by weight fenofibrate; a polyethylene glycol, especially polyethylene glycol 6000 at about 7% to about 13% by weight; and a polyethylene-polypropylene glycol, especially poloxamer 407 at about 7% to about 13% by weight; wherein the combination is deposited by sublimation of a sublimable carrier, especially menthol, from a solid solution that comprises fenofibrate, the polyethylene glycol, the polyethylene-polypropylene glycol, and the sublimable carrier. The composition can also include a pharmaceutical disintegrant selected from the group consisting of crospovidone, a crosslinked carboxymethylcellulose salt (especially crosslinked carboxymethylcellulose sodium), the bicarbonate or carbonate salts; especially alkali metal bicarbonates or carbonates like sodium bicarbonate; the organic carboxylic acids, especially citric acid, tanic acid, ascorbic acid, benzoic acid, citric acid, fumaric acid, lactic acid, malic acid, sorbic acid, and tartaric acid; and combinations of any of the foregoing.

In still yet a further aspect, the present invention relates to a solid oral dosage form that includes a pharmaceutical composition having about 145 mg of fenofibrate that has been deposited on a plurality of particles of microcrystalline cellulose by sublimation of a sublimable carrier from a solid solution comprising fenofibrate and the sublimable carrier; wherein in human *in vivo* pharmacokinetic studies in which the dosage form is administered in the fasted state, the area under the 48-hour AUC curve (AUC₄₈) is about 121367 h*ng/g to about 287539 h*ng/g; the area under the AUC curve extrapolated to infinite time (AUC_{∞}) is about 134750 h*ng/g to about 345390 h*ng/g; and the maximum plasma concentration (Cₘₐₓ) is about 6357 ng/g to about 14627 ng/g. This solid oral dosage, in certain of its detailed aspects, exhibits an average AUC₄₈ of about 175335 h*ng/g, an average AUC_{∞} of about 213652 h*ng/g, and an average Cₘₐₓ of about 10570 ng/g.

### DETAILED DESCRIPTION OF THE INVENTION

In one embodiment, the present invention provides a pharmaceutical composition that includes non-mechanically micronized microparticles of fenofibrate, a polyethylene glycol, and a polyethylene-polypropylene glycol.

Non-mechanically micronized microparticles have mean dimensions of about 0.1 µm to about 10 µm and are produced by non-mechanical comminution techniques. Non-mechanical comminution techniques are techniques other than milling (ball, impingement, high energy), spray drying, and high-pressure homogenization. For purposes of the present application, the technique of lyophilization is considered a mechanical micronization technique and, hence, microparticles produced by lyophilization are excluded from non-mechanically micronized microparticles. Particle size measurement is well-known to the skilled artisan and can be accomplished by, for example, the well-known technique of laser light-scattering (Malvern Company).

The non-mechanically micronized microparticles of fenofibrate of the present invention can be obtained by, for example, the technique of sublimation micronization. Microparticles so obtained are referred to as "sublimation micronized" microparticles and the material of which such microparticle are comprised is referred to as "sublimation micronized". The technique of sublimation micronization is described in published United States Patent Application US 2003/0224059 (Lerner et al.).

The microparticles of fenofibrate of the present invention are obtained via sublimation micronization by removing a sublimable carrier from a solid solution of fenofibrate in the sublimable carrier. The fenofibrate can be present with the sublimable carrier in the solid solution as discrete molecules, or it can be present in aggregates of a few hundred, a few thousand, or more molecules. The drug need only be dispersed on a sufficiently small scale so that sufficiently small, discrete microparticles are ultimately obtained. Preferably, the fenofibrate in the solid solution is dissolved in the sublimable carrier.

Sublimable carriers have a measurable vapor pressure below their melting point. Preferred sublimable carriers have a vapor pressure of at least about 10 Pascal, more preferably at least about 50 Pascal at about 10° C or more below their normal melting points. Preferably, the sublimable carrier has a melting point between about -10° C and about 200°C, more preferably between about 20° C and about 60° C, most preferably between about 40°C and about 50° C. Preferably, the sublimable carrier is a substance that is classified by the United States Food and Drug Administration as generally recognized as safe (i.e., GRAS). Examples of suitable sublimable carriers include menthol, thymol, camphor, t-butanol, trichloro-t-butanol, imidazole, coumarin, acetic acid (glacial), dimethylsulfone, urea, vanillin, camphene, salicylamide, and 2-aminopyridine. Menthol is a particularly preferred sublimable carrier.

The microparticles of the present invention are formed by removal of sublimable carrier from a solid solution, made as described above, at a temperature below the melting point of the solid solution. The solid solution must be kept at a temperature below its melting point to preserve the solid solution during the process of removing the sublimable carrier. The sublimable carrier can be removed from the solid solution by, for example, treating the solid solution, deposited on a pharmaceutical carrier particle where applicable as discussed *infra,* in a stream of air, preferably heated air, in, for example, a fluidized bed drier.

The pharmaceutical compositions of the present invention further include polyalkylene glycols. Preferably the pharmaceutical compositions of the present invention include at least one polyethylene glycol (PEG) and at least one polyethylene-polypropylene glycol.

Polyethylene glycols useful in the practice of the present invention have the general formula -(-CH₂-CH₂-O-)ₓ- and can be characterized by the arithmetic mean value of X (<X_{N}>) or the molecular weight corresponding thereto as described in, for example, *Polyethylene Glycols,* 23 National Formulary, 3052 (United States Pharmacopeial Convention, 2005). Polyethylene glycol 6000 is a preferred polyethylene glycol for use in the practice of the present invention.

Polyethylene-polypropylene glycols useful in the practice of the present invention have the general structure -(O-CH₂CH₂-)ₐ-O-(-CH(CH₃)CH₂-)_{b}-(-O-CH₂CH₂-)ₐ-OH and are commonly referred to as "poloxamers". Preferred poloxamers for use in the practice of the present invention are described in the monograph of like name in the U.S. National Formulary. *Poloxamers,* 23 National Formulary, 3051 (United States Pharmacopeial Convention, 2005). The polyethylene - polypropylene glycol commonly designated "poloxamer 407" is a particularly preferred polyethylene- polypropylene glycol for use in the practice of the present invention.

The pharmaceutical compositions of the present invention can be and in preferred embodiments are deposited on a plurality of pharmaceutical carrier particles. Pharmaceutical carrier particles useful as support, substrate, or carrier for the pharmaceutical formulation of the present invention are made of comestible substances and are well known in the art. Examples of useful pharmaceutical carrier particles include particles, that can be non-pariel pellets, typically between about 0.1 mm. and about 2 mm. in diameter, and made of, for example, starch, particles of microcrystalline cellulose, lactose particles or, particularly, sugar particles. Suitable sugar particles (pellets, e.g. non-pariel 103, Nu-core, Nu-pariel) are commercially available in sizes from 35 to 40 mesh to 18 to 14 mesh. Preferred pharmaceutical carrier particles are made of non-hydrosoluble material, for example microcrystalline cellulose. Carrier particles comprised of microcrystalline cellulose (e.g. Avicel^{®}) are particularly preferred pharmaceutical carrier particles. The skilled artisan knows other pellets or spheres useful as pharmaceutical carrier particles.

Pharmaceutical compositions according to the present invention can be made by combining fenofibrate, polyethylene glycol, polyethylene- polypropylene glycol, and a sublimable carrier. The above components can be combined neat or, in embodiments in which the composition is deposited on a plurality of pharmaceutical carrier particles, together with a suitable solvent. Suitable solvents dissolve fenofibrate, polyethylene glycol, polyethylene - polypropylene glycol, and the sublimable carrier, but do not dissolve pharmaceutical carrier particles and further are chemically inert to any of the components, and can be readily removed at a convenient temperature, especially a temperature < 100° C, optionally with the aid of an applied vacuum. Ethanol is an example of a suitable solvent.

The combination of components are combined and warmed to form a homogeneous mixture, preferably a solution, and cooled to obtain a solid solution. The fenofibrate can be present with the sublimable carrier in the solid solution as discrete molecules, or it can be present in aggregates of a few hundred, a few thousand, or more molecules. The drug need only be dispersed on a sufficiently small scale so that sufficiently small, discrete microparticles are ultimately obtained.

Preferably, the drug in the solid solution is dissolved in the sublimable carrier. In embodiments in which the sublimation micronized microparticles are deposited on a plurality of pharmaceutical carrier particles, the warm solution of components in sublimable carrier is combined with pharmaceutical carrier particles, for example by mixing, and the combination allowed to cool to form the solid solution on the pharmaceutical carrier particles. Alternatively, pharmaceutical carrier particles are combined with a solution of sublimable carrier, fenofibrate, polyethylene glycol, and polyethylene - polypropylene glycol in a suitable solvent (e.g. ethanol). The solvent is removed, optionally with the aid of applied heat and vacuum, to obtain pharmaceutical carrier particles having deposited thereon a solid solution of the fenofibrate, polyethylene glycol, and polyethylene - polypropylene glycol in the sublimable carrier (e.g. menthol).

After formation of the solid solution, whether deposited on pharmaceutical carrier particles or not, the pharmaceutical formulations of the present invention are subsequently formed by removal of sublimable carrier from the solid solution, made as described above, at a temperature below the melting point of the solid solution. The solid solution must be kept at a temperature below its melting point to preserve the solid solution during the process of removing the sublimable carrier. The sublimable carrier can be removed from the solid solution by, for example, treating the solid solution, deposited on a pharmaceutical carrier particle where applicable, in a stream of air, preferably heated air, in, for example, a fluidized bed drier.

In preferred embodiments, removal of the sublimable carrier results in formation of non-mechanically micronized microparticles of fenofibrate, which microparticles can further contain at least a portion of the polyethylene glycol and polyethylene-polypropylene glycol. Furthermore, at least a portion of the fenofibrate can be in solution or intimately associated with either or both of the polyethylene glycol and polyethylene-polypropylene glycol that are not necessarily with the non-mechanically micronized microparticles.

Applicants' invention is not limited by a particular theory of operation. But applicants believe that, after removal of the sublimable carrier, at least a portion of the fenofibrate is dissolved in or intimately associated with the polyalkylene glycols. The expression "intimately associated" excludes a simple physical mixture such as can be achieved by, for example, dry blending, dry granulation, or wet granulation in the presence of a liquid that does not at least partially dissolve the components.

The pharmaceutical compositions of the present invention, particularly when deposited on a plurality of pharmaceutical carrier particles, are well suited for manufacture of liquid and especially solid oral dosage forms such as compressed tablets and filled capsules. In another embodiment, the present invention provides oral dosage forms, especially solid oral dosage forms, preferably compressed tablets, that include the pharmaceutical compositions of the present invention.

Compressed tablets are formulated from pharmaceutical compositions containing the microparticles of the pharmacologically active substance or drug, or using pharmaceutical carrier particles bearing such microparticles, and pharmacologically inert (pharmaceutically acceptable) additives or excipients.

For making a tablet, it will typically be desirable to include one or more benign pharmaceutical excipients in the pharmaceutical composition. The pharmaceutical composition of the present invention may contain one or more diluents added to make the tablet larger and, hence, easier for the patient and caregiver to handle. Common diluents are microcrystalline cellulose (e.g. Avicel^{®}), microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol and talc.

Binders also may be included in tablet formulations to help hold the tablet together after compression. Some typical binders are acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e,g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate and starch.

The tablet may further include a disintegrant to accelerate disintegration of the tablet in the patient's stomach. Disintegrants include alginic acid, carboxymethyl cellulose calcium, carboxymethylcellulose sodium, colloidal silicon dioxide, crosslinked carboxymethylcellulose sodium or calcium (croscarmellose sodium (e.g. Ac-Di-Sol^{®}, Primellose^{®} or croscarmelose calcium), crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

In addition to or in place of alginic acid, other organic carboxylic acids can be included in the formulation. The organic acids include tannic acid, citric acid, fumaric acid tartaric acid, lactic acid, malic acid, ascorbic acid, benzoic acid, sorbic acid, and the like. Tannic acid and citric acid are particularly preferred organic carboxylic acids for use in this and other embodiments of the present invention.

The pharmaceutical compositions of the present invention can, and in preferred embodiments do contain a bicarbonate or carbonate, especially an alkali metal bicarbonate or carbonate. Examples of preferred alkali metal carbonates and bicarbonates include sodium bicarbonate, sodium carbonate, potassium bicarbonate, potassium carbonate. Alkailne earth metal carbonates like calcium carbonate and magnesium carbonate can also be used.

A pharmaceutical composition for making compressed tablets may further include glidants, lubricants, flavorings, colorants and other commonly used excipients.

Pharmaceutical carrier particles bearing microparticles of a drug made in accordance with the present invention have excellent bulk flow properties and can be used directly, alone or in combination with carrier particles that do not carry a drug, to make capsule dosage forms. If necessary, diluents such as lactose, mannitol, calcium carbonate, and magnesium carbonate, to mention just a few, can be formulated with the microparticle-bearing pharmaceutical carrier particles when making capsules

Liquid oral pharmaceutical compositions of the present invention comprise microparticles or microparticle-bearing pharmaceutical carrier particles and a liquid carrier such as water, vegetable oil, alcohol, polyethylene glycol, propylene glycol or glycerin, most preferably water.

Liquid oral pharmaceutical compositions may contain emulsifying agents to disperse uniformly throughout the composition the active ingredient, drug delivery vehicle, or excipient having low solubility in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid oral pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

The liquid oral pharmaceutical composition also may contain sweetening agents, such as sorbitol, saccharin, sodium saccharin, sucrose, aspartame, fructose, mannitol and invert sugar; preservatives and chelating agents such as alcohol, sodium benzoate, butylated hydroxy toluene, butylated hydroxyanisole and ethylenediamine tetraacetic acid; and buffers such as guconic acid, lactic acid, citric acid or acetic acid, sodium gluconate, sodium lactate, sodium citrate or sodium acetate.

Solid oral dosage forms formulated and compounded with the sublimation micronized microparticles of fenofibrate together with a polyethylene glycol and a polyethylene-polypropylene glycol, prepared as hereinabove described, provide for improved bioavailability of fenofibrate as demonstrated by both *in vitro* dissolution (release) and human *in vivo* pharmacokinetic (plasma concentration) testing. The results of both *in vivo* and *in vitro* testing disclosed herein were obtained with tablets containing about 145 mg fenofibrate and having a nominal weight of 792 mg each.

Time-dependent *in vitro* release (dissolution) profiles disclosed herein were obtained at 37°C using a USP Type-II dissolution tester operating at 50 rpm and filled with 1000 mL of 0.5 wt-% sodium lauryl sulfate in water. The concentration of fenofibrate in the test liquid was determined by HPLC.

Pharmacokinetic data disclosed herein were obtained in human *in vivo* experiments by determining the blood plasma concentration of the metabolite, fenofibric acid, as a function of time to afford a well-know Boltzmann-shaped cumulative plasma concentration (AUC) curve. Individual points are reported with reference to selected actual or extrapolated time points on the AUC curve.

Thus, the area under the 48-hour AUC curve, AUC₄₈, refers to the cumulative blood concentration up to the 48 hour time point (the last point measured). AUC_{∞} refers to the area under the AUC curve extrapolated to infinite time. Cₘₐₓ refers to the maximum absolute plasma concentration measured in the 48 hour test (i.e. the maximum point on the 48-hour AUC curve). Average AUC (<AUC>) is the arithmetic average plasma concentration of fenofibric acid measured over the course of the plasma concentration measurement period (about 48 hours).

The present invention, in certain of its embodiments, is illustrated by the following non-limiting examples. In the following examples, fasted state means that the subject has not taken food within the ten hours preceding dosing. Fed state means that the subject has taken food about one-half hour prior to dosing.

### EXPERIMENTAL

### A. Fenofibrate granulate

Menthol (1.333Kg) was melted in a glass reactor at 50 °C, with stirring. Fenofibrate (133.3 gm), poloxamer 407 (Lutrol F127, 76 gm), and polyethylene glycol 6000 (76 gm) were charged to the reactor. The menthol melt was stirred at 50°C until all the components had dissolved. Microcrystalline cellulose (Avicel PH 101, 106.7 gm) was added to the melt, which was stirred until a uniform suspension was obtained.

The menthol melt was divided into three equal portions and poured into three trays (stainless steel, 0.133 m² each) that were cooled to -40°C for quick solidification of the menthol suspension. The solid material on the trays was removed and coarsely milled through a 2.5 mm screen using an Erweka mill. The obtained powder was again divided into three portions and returned to the trays. Menthol was removed from the material on the trays by sublimation in a high vacuum tray drier at 0.2 mbar and 36°C for about 53 hours. The resulting powder was removed from the trays and milled through a 1.6 mm screen using an Erweka mill so as to not effect substantial comminution of the already-formed particles. The granulate so obtained was weighed (346.4 gm) for a yield of 88%.

### B. Fenofibrate Tablets (145 mg)-

The Fenofibrate granulate from step A was milled through a 0.8 mm screen using an Erweka mill. The milled granulate (336 gm) was added to a polyethylene bag (50 x 70 cm). Crospovidone (108 gm), sodium bicarbonate (72 gm) and citric acid anhydrous (72 gm) were added and the blend mixed for 5 minutes. Magnesium stearate (12 gm) was added to the bag and the blend mixed for a further 1/2 minute. The total amount of blend so obtained was 600 grams.

The blend was compressed into tablets on a Manesty F3 single punch tabletting machine using oval shaped (8.8 mm x 17.6 mm) normal concave punches. Tablet design weight was 785 mg ± 39.3 mg at a hardness of 5 - 7 Kp. The tablets obtained had an average weight of 792 mg and a hardness of 6 Kp. Several batches were made and labeled MAZ 149B, MAZ149B1 and MAZ149B2, respectively.

### C In vitro release

The release (dissolution) of fenofibrate from the tablets was tested using a USP type II dissolution tester filled with 1000 ml 0.5% sodium lauryl sulfate (SLS) (w/v) in water at 37°C and 50 revolutions per minute (rpm). The amount of fenofibrate in each sample was determined by HPLC as above. The results are given in tables C1-C3 for three batches.

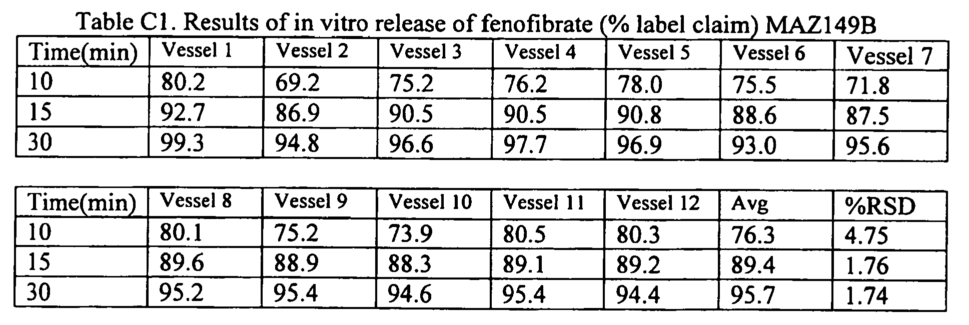

**Table C2. Results of in vitro release of fenofibrate (% label claim) MAZ149B1**

| Time(min) | Vessel 1 | Vessel 2 | Vessel 3 | Vessel 4 | Vessel 5 | Vessel 6 | Avg | %RSD |
|---|---|---|---|---|---|---|---|---|
| 10 | 58.6 | 55.8 | 51.4 | 55.2 | 51.5 | 61.5 | 55.7 | 7.11 |
| 15 | 79.5 | 77.3 | 73.6 | 77.5 | 74.4 | 79.2 | 76.9 | 3.16 |
| 30 | 90.7 | 88.9 | 86.5 | 88.5 | 89.1 | 89.5 | 88.9 | 1.56 |

**Table C3. Results of in vitro release of fenofibrate (% label claim) MAZ149B2**

| Time(min) | Vessel 1 | Vessel 2 | Vessel 3 | Vessel 4 | Vessel 5 | Vessel 6 | Avg | %RSD |
|---|---|---|---|---|---|---|---|---|
| 10 | 69.0 | 66.3 | 68.9 | 71.0 | 72.4 | 63.8 | 68.6 | 4.54 |
| 15 | 80.2 | 79.6 | 81.1 | 81.1 | 81.0 | 77.6 | 80.1 | 1.72 |
| 30 | 86.9 | 87.8 | 88.0 | 87.1 | 87.1 1 | 86.9 | 87.3 | 0.56 |

### D. In vivo pharmacokinetic trial

Pharmacokinetic Test of MAZ149B and TriCor^{®} 145 mg (NDA # 19-304/S-005).

A four way crossover bioequivalence pharmacokinetic trial was carried out in 12 healthy volunteers using MAZ149B (145 mg, described *supra*) and TriCor^{®} (145 mg) as two of the test arms. The other two arms were other test formulations prepared according to the present invention. A one week washout was taken between each arm of the test. Blood samples were taken at 0, 1, 2, 3, 3.5, 4, 4.5, 5, 5.5, 6, 6.5, 7, 8, 9, 10, 12, 16, 24 and 48 hours (19 samples per trial) and analyzed for fenofibric acid by a validated method. The 4 arm trials were carried out in both the fasted and fed states.

### Results

Fasted-state data was obtained for volunteers 1- 11 for the test MAZ 149B (N=11) and for volunteers 2-11 for the reference TriCor^{®} (N-10). The results are collected in table D1. The average values showed the bioavailability of the test to be 97.4% of the reference based on AUC₄₈ (175334 vs. 180010 h*ng/g) and 97.7% of the reference based on AUC_{∞} (213653 vs. 218628 h*ng/g). The corresponding geometric mean values showed 97.5% based on AUC₄₈ (169481 vs. 173880 h*ng/g) and 97.5% based on AUC_{∞} (205217 vs. 210558 h*ng/g). The geometric mean of the ratio of the individual ratios of test to reference AUC_{∞} was 1.006. The average values for Cₘₐₓ showed the test to be 99% of the reference (10570 vs. 10624 ng/g) and the geometric mean to be 100.7% (10340 vs. 10270 ng/g). The geometric mean of the ratios of the test to reference of the individual volunteers was 1.021. The variability of the bioavailability was very similar 28.95% vs. 27.16% for %CV (variation in the variable) of the AUC₄₈ values. The average terminal half life (terminal half-life for elimination) was 20.0 hours for the test product and 19.9 hours for the reference while the average Tₘₐₓ was 2.5 hours for the test and 2.1 hours for the reference. One can conclude that the two formulations are bioequivalent in the fasted state.

Fed-state data was obtained for volunteers 1-5, 7-10 and 12 (N=10) for both the test MAZ149B and the TriCor^{®} reference product. The results are collected in table D2. The average values showed the bioavailability of the test to be107.1 % of the reference based on AUC₄₈ (150511 vs. 140627 h*ng/g) and 112.0% of the reference based on AUC_{∞} (185149 vs. 165310 h*ng/g). The corresponding geometric mean values showed 106.8% based on AUC₄₈ (145402 vs. 136134 h*ng/g) and 111.2% based on AUC_{∞} (174021 vs. 156459 h*ng/g). The geometric mean of the ratio of the individual ratios of test to reference AUC_{∞} was 1.112. The average values for Cₘₐₓ showed the test to be 79.0 % of the reference (7557 vs.9567 ng/g) and the geometric mean to be 77.5% (7147 vs. 9217 ng/g). The geometric mean of the ratios of the test to reference of the individual volunteers was 0.775. The variability of the bioavailability was very similar 27.16% vs. 26.41% for %CV (variation in the value) of the AUC₄₈ values. The average terminal half life was 17.4 hours for the test product and 16.1 hours for the reference while the average Tmax was 8.0 hours for the test and 3.6 hours for the reference. The improved bioavailability coupled to a lower Cₘₐₓ and the later T*max* indicate an improved product in the fed state.

Further aspects and features of the present inventions are set out in the following numbered clauses.
1. A pharmaceutical composition comprising non-mechanically micronized microparticles of fenofibrate, polyethylene glycol, and polyethylene-polypropylene glycol.
2. The pharmaceutical composition of clause 1 wherein the non-mechanically micronized microparticles of fenofibrate are made by sublimation micronization.
3. The pharmaceutical composition of any preceding clause wherein the non-mechanically micronized microparticles are deposited on a plurality of pharmaceutical carrier particles.
4. The pharmaceutical composition of clause 3 wherein menthol is the sublimable carrier in the sublimation micronization step.
5. The pharmaceutical composition of any preceding clause wherein the polyethylene glycol is polyethylene glycol 6000
6. The pharmaceutical composition of any preceding clause wherein the polyethylene-polypropylene glycol is poloxamer 407.
7. The pharmaceutical composition of clause 6 wherein the pharmaceutical composition is in the form of a solid oral dosage form comprising about 15% to about 25% by weight fenofibrate, about 7% to about 13% poloxamer 407, and about 7% to about 13% polyethylene glycol 6000.
8. The pharmaceutical composition any preceding clause further comprising a pharmaceutical disintegrant selected from the group consisting of crospovidone, croscarmellose sodium, the bicarbonate or carbonate salts, the organic carboxylic acids, and combinations of any of the foregoing.
9. The pharmaceutical composition of clause 8 wherein the organic carboxylic acids are selected from citric acid, tanic acid, ascorbic acid, benzoic acid, fumaric acid, lactic acid, malic acid, sorbic acid, and tartaric acid, especially citric acid and tartaric acid.
10. The pharmaceutical composition of clause 9 comprising about 12% by weight of either citric acid or tartaric acid, especially about 12% by weight of citric acid.
11. The pharmaceutical composition of clause 8 wherein the bicarbonate or carbonate salts are alkali metal bicarbonates or carbonates such as sodium bicarbonate, sodium carbonate, potassium bicarbonate, potassium carbonate, calcium carbonate, magnesium carbonate, especially sodium bicarbonate.
12. The pharmaceutical composition of clause 11 comprising about 12% by weight of sodium bicarbonate.
13. The pharmaceutical composition of any preceding clause further comprising microcrystalline cellulose, particularly about 15% by weight of microcrystalline cellulose.
14. The pharmaceutical composition of any preceding clause further comprising crosspovidone, particularly about 18% by weight of crosspovidone.
15. The pharmaceutical composition of any preceding clause in solid oral dosage form, especially a compressed tablet.
16. The solid oral dosage form of clause 15 having a time-dependent *in vitro* fenofibrate release profile such that at least about 51 % by weight of the fenofibrate is released in about 10 minutes, at least about 73% by weight of the fenofibrate is released in about 15 minutes, and at least about 85% by weight of the fenofibrate is released in about 30 minutes.
17. The solid oral dosage form of clause 15 having a time-dependent *in vitro* release profile such that about 51 % to about 81 % by weight of the fenofibrate is released in about 10 minutes, about 73% to about 93% by weight of the fenofibrate is released in about 15 minutes, and about 85% by weight to about all of the fenofibrate is released in about 30 minutes.
18. A solid oral dosage form comprising a pharmaceutical composition comprising about 145 mg of sublimation micronized fenofibrate wherein in human *in vivo* pharmacokinetic studies in which the dosage form is administered in the fasted state, the area under the 48-hour AUC curve (AUC₄₈) is about 121367 h*ng/g to about 287539 h*ng/g; the area under the AUC curve extrapolated to infinite time (AUC_{∞}) is about 134750 h*ng/g to about 345390 h*ng/g; and the maximum plasma concentration (Cₘₐₓ) is about 6357 ng/g to about 14627 ng/g.
19. The solid oral dosage form of clause 18 wherein, in human *in vivo* pharmacokinetic studies in which the dosage form is administered in the fasted state, the average AUC₄₈ is about 175335 h*ng/g, the average AUC_{∞} is about 213652 h*ng/g, and the average Cₘₐₓ is about 10570 ng/g.
20. A solid oral dosage form comprising a pharmaceutical composition comprising about 145 mg of sublimation micronized fenofibrate wherein in human *in vivo* pharmacokinetic studies in which the dosage form is administered in the fed state, the area under the 48-hour AUC curve (AUC₄₈) is about 91601 h*ng/g to about 217512 h*ng/g; and the area under the AUC curve extrapolated to infinite time (AUC_{∞}) is about 97182 h*ng/g to about 308070 h*ng/g.
21. The solid oral dosage form of clause 17 wherein the average AUC₄₈ is about 150511 h*ng/g and the average AUC_{∞} is about 185149 h*ng/g.
22. The solid oral dosage form of any of clauses 16 to 21 wherein the solid oral dosage form is a compressed tablet.
23. A pharmaceutical composition comprising a plurality of pharmaceutical carrier particles having deposited thereon a combination of fenofibrate, a polyethylene glycol, and a polyethylene-polypropylene glycol, wherein the combination is deposited by sublimation of a sublimable carrier from a solid solution that comprises fenofibrate, the polyethylene glycol, the polyethylene-polypropylene glycol, and the sublimable carrier.
24. The pharmaceutical composition of clause 23 wherein the sublimable carrier is menthol.
25. The pharmaceutical composition of any of clauses 23 to 24 wherein the polyethylene glycol is polyethylene glycol 6000.
26. The pharmaceutical composition of any of clauses 23 to 25 wherein the polyethylene-polypropylene glycol is poloxamer 407.
27. The pharmaceutical composition of any of clauses 23 to 26 wherein the pharmaceutical composition is in the form of a solid oral dosage form comprising about 15% to about 25% by weight fenofibrate, about 7% to about 13% poloxamer 407, and about 7% to about 13% polyethylene glycol 6000.
28. The pharmaceutical composition of any of clauses 23 to 26 further comprising a pharmaceutical disintegrant selected from the group consisting of crospovidone, croscarmellose sodium, the bicarbonate or carbonate salts, the organic carboxylic acids, and combinations of any of the foregoing.
29. The pharmaceutical composition of clause 28 wherein the organic carboxylic acids are selected from citric acid, tanic acid, ascorbic acid, benzoic acid, fumaric acid, lactic acid, malic acid, sorbic acid, and tartaric acid, especially citric acid and tartaric acid.
30. The pharmaceutical composition of clause 29 comprising about 12% by weight of either citric acid or tartaric acid, especially about 12% by weight of citric acid.
31. The pharmaceutical composition of clause 28 wherein the bicarbonate or carbonate salts are alkali metal bicarbonates or carbonates such as sodium bicarbonate, sodium carbonate, potassium bicarbonate, potassium carbonate, calcium carbonate, magnesium carbonate, especially sodium bicarbonate.
32. The pharmaceutical composition of clause 31 comprising about 12% by weight of sodium bicarbonate.
33. The pharmaceutical composition of any of clauses 23 to 32 further comprising microcrystalline cellulose, particularly about 15% by weight of microcrystalline cellulose.
34. The pharmaceutical composition of any of clauses 23 to 33 further comprising crosspovidone, particularly about 18% by weight of crosspovidone.
35. The pharmaceutical composition of any of clauses 23 to 34 in solid oral dosage form, especially a compressed tablet.
36. A solid oral dosage form comprising a pharmaceutical composition comprising about 15% to about 25% by weight of fenofibrate, about 7% to about 13% by weight poloxamer 407, about 7% to about 13% polyethylene glycol 6000, about 15% by weight microcrystalline cellulose, about 18% by weight crosspovidone, about 12% by weight sodium bicarbonate, and about 12% by weight of either citric acid or tartaric acid, wherein at least the fenofibrate, the poloxamer 407, and the polyethylene glycol 6000 are deposited on the microcrystalline cellulose by sublimation of a sublimable carrier from a solid solution of at least the fenofibrate, the poloxamer 407, and the polyethylene glycol 6000 with the sublimable carrier.
37. The solid oral dosage form of clause 36 comprising about 12% by weight citric acid.
38. The solid oral dosage form of clause 36 or 37 having a time-dependent *in vitro* fenofibrate release profile such that at least about 51% by weight of the fenofibrate is released in about 10 minutes, at least about 73% by weight of the fenofibrate is released in about 15 minutes, and at least about 85% by weight of the fenofibrate is released in about 30 minutes.
39. The solid oral dosage form of clause 36 or 37 having a time-dependent in vitro release profile such that about 51% to about 81% by weight of the fenofibrate is released in about 10 minutes, about 73% to about 93% by weight of the fenofibrate is released in about 15 minutes, and about 85% by weight to about all of the fenofibrate is released in about 30 minutes.
40. A solid oral dosage form comprising a pharmaceutical composition comprising about 145 mg of fenofibrate that has been deposited on a plurality of particles of microcrystalline cellulose by sublimation of a sublimable carrier from a solid solution comprising fenofibrate and the sublimable carrier; wherein in human *in vivo* pharmacokinetic studies in which the dosage form is administered in the fasted state, the area under the 48-hour AUC curve (AUC₄₈) is about 121367 h*ng/g to about 287539 h*ng/g; the area under the AUC curve extrapolated to infinite time (AUC_{∞}) is about 134750 h*ng/g to about 345390 h*ng/g; and the maximum plasma concentration (Cₘₐₓ) is about 6357 ng/g to about 14627 ng/g.
41. The solid oral dosage form of clause 40 wherein, in human *in vivo* pharmacokinetic studies in which the dosage form is administered in the fasted state, the average AUC₄₈ is about 175335 h*ng/g, the average AUC_{∞} is about 213652 h*ng/g, and the average Cₘₐₓ is about 10570 ng/g.
42. A solid oral dosage form comprising a pharmaceutical composition comprising about 145 mg of fenofibrate that has been deposited on a plurality of particles of microcrystalline cellulose by sublimation of a sublimable carrier from a solid solution comprising fenofibrate and the sublimable carrier; wherein in human *in vivo* pharmacokinetic studies in which the dosage form is administered in the fed state, the area under the 48-hour AUC curve (AUC₄₈) is about 91601 h*ng/g to about 217512 h*ng/g; and the area under the AUC curve extrapolated to infinite time (AUC_{∞}) is about 97182 h*ng/g to about 308070 h*ng/g.
43. The solid oral dosage form of clause 42 wherein the average AUC48 is about 150511 h*ng/g and the average AUC_{∞} is about 185149 h*ng/g.
44. The solid oral dosage form of any of clauses 36 to 44 wherein the solid oral dosage form is a compressed tablet.

**Table D1 (Fasted State)**

| | RESULTS OF | fenofibric acid fasted state 10536004-MAZ149B vs. TriCor® | | | | dose test (mg)= | 145 | | |
|---|---|---|---|---|---|---|---|---|---|
| | | | | | | dose ref (mg) = | 145 | | |
| volunteer | AUC₄₈ (h*ng/g) | AUCinf (h*ng/g) | t1/2 | | | Tmax (h) | Cmax (ng/g) | **Cmaxtest/Cmaxref** | **AUCinftest/AUCinfref** |
| 1 (test) | 133053.0 | 149267.0 | 15.5 | | | 3.0 | 9010.0 | | |
| 2 (test) | 166526.0 | 189578.0 | 17.1 | | | 2.0 | 12699.0 | 1.04 | 0.79 |
| 3 (test) | 142995.0 | 193559.0 | 25.8 | | | 2.0 | 6357.0 | 0.91 | 0.94 |
| 4 (test) | 121367.0 | 134750.0 | 15.4 | | | 2.0 | 9360.0 | 0.95 | 1.11 |
| 5 (Test) | 156850.0 | 176988.0 | 16.8 | | | 2.0 | 10489.0 | 0.87 | 0.92 |
| 6 (test) | 142581.0 | 167086.0 | 18.1 | | | 1.5 | 8397.0 | 1.39 | 1.09 |
| 7 (test) | 142733.0 | 201134.0 | 28.6 | | | 4.5 | 10952.0 | 1.15 | 1.12 |
| 8 (test) | 209259.0 | 251532.0 | 19.7 | | | 1.5 | 11138.0 | 0.74 | 0.94 |
| 9 (test) | 191656.0 | 227307.0 | 18.3 | | | 3.0 | 11300.0 | 1.13 | 1.05 |
| 10 (test) | 287539.0 | 345390.0 | 19.2 | | | 5.0 | 14627.0 | 1.09 | 1.16 |
| 11 (test) | 234120.0 | 313588.0 | 25.1 | | | 1.5 | 11944.0 | 1.08 | 1.01 |
| 12 (test) | | | | | | | | | |
| | | | | | | | | | |
| 1 (ref) | | | | | | | | | |
| 2 (ref) | 199790.0 | 239900.0 | 19.2 | | | 3.0 | 12242.0 | | |
| 3 (ref) | 150941.0 | 206776.0 | 25.8 | | | 1.5 | 6966.0 | | |
| 4 (ref) | 113223.0 | 121099.0 | 13.1 | | | 2.0 | 9894.0 | | |
| 5 (ref) | 166239.0 | 193362.0 | 17.5 | | | 4.0 | 12085.0 | | |
| 6 (ref) | 117039.0 | 153347.0 | 23.7 | | | 2.0 | 6020.0 | | |
| 7 (ref) | 151310.0 | 179099.0 | 18.7 | | | 1.0 | 9537.0 | | |
| 8 (ref) | 218602.0 | 267224.0 | 21.4 | | | 1.5 | 15025.0 | | |
| 9 (ref) | 188187.0 | 217502.0 | 16.7 | | | 2.5 | 10026.0 | | |
| 10 (ref) | 257562.0 | 298831.0 | 18.4 | | | 1.5 | 13396.0 | | |
| 11 (ref) | 237204.0 | 309136.0 | 24.1 | | | 2.0 | 11052.0 | | |
| 12 (ref) | | | | | | | | | |
| AVG(test) | 175334.5 | 213652.6 | 20.0 | | | 2.5 | 10570.3 | 1.035 | 1.012 |
| AVG (ref) | 180009.7 | 218627.6 | 19.9 | | | 2.1 | 10624.3 | | |
| | | | | | | | | | |
| Geomn(test) | 169481.3 | 205216.6 | 19.5 | | | 2.3 | 10339.7 | 1.021 | 1.006 |
| Geomn(ref) | 173879.9 | 210558.3 | 19.5 | | | 2.0 | 10270.3 | | |
| | | | | | | | | | |
| stddev(test) | 50758 | 66323 | 4.48 | | | 1.21 | 2241.80 | 0.18 | 0.12 |
| stddev(ref) | 48896 | 61060 | 3.88 | | | 0.88 | 2761.61 | | |
| | | | | | | | | | |
| %CV (test) | 28.95% | 31.04% | 0.22 | | | 0.48 | 0.21 | | |
| %CV (ref) | 27.16% | 27.93% | 0.20 | | | 0.42 | 0.26 | | |

**Table D2 (Fed State)**

| | RESULTS OF | fenofibric acid fed state 10536005-MAZ149B vs. TriCor® | | | | dose test (mg)= | 145 | | |
|---|---|---|---|---|---|---|---|---|---|
| | | | | | | dose ref (mg) = | 145 | | |
| volunteer | AUC₄₈ (h*ng/g) | AUC₄₈inf (h*ng/g) | t1/2 | | | Tmax (h) | Cmax (ng/g) | **Cmaxtest/ Cmaxref** | **AUCInftest/ AUCinfref** |
| 1 (test) | 151598.0 | 201715.0 | 23.6 | | | 5.5 | 6517.0 | 1.01 | 1.15 |
| 2 (test) | 119863.0 | 126328.0 | 11.8 | | | 2.0 | 10583.0 | 0.87 | 1.10 |
| 3 (test) | 91601.0 | 97182.0 | 10.5 | | | 9.0 | 5224.0 | 0.76 | 1.05 |
| 4 (test) | 159339.0 | 202949.0 | 20.2 | | | 5.0 | 6725.0 | 0.86 | 1.14 |
| 5 (Test) | 183511.0 | 217445.0 | 18.6 | | | 3.5 | 14538.0 | 1.06 | 1.10 |
| 6 (test) | | | | | | | | | |
| 7 (test) | 106779.0 | 115154.0 | 11.9 | | | 4.5 | 5250.0 | 0.62 | 1.06 |
| 8 (test) | 195150.0 | 251495.0 | 19.9 | | | 12.0 | 7083.0 | 0.65 | 0.95 |
| 9 (test) | 119423.0 | 129598.0 | 12.8 | | | 4.5 | 6285.0 | 0.60 | 1.11 |
| 10 (test) | 217512.0 | 308070.0 | 24.1 | | | 10.0 | 8236.0 | 0.66 | 1.32 |
| 11 (test) | | | | | | | | | |
| 12 (test) | 160332.0 | 201554.0 | 20.6 | | | 24.0 | 5126.0 | 0.81 | 1.18 |
| | | | | | | | | | |
| 1 (ref) | 144704.0 | 175441.0 | 17.4 | | | 5.5 | 6441.0 | | |
| 2 (ref) | 110211.0 | 115275.0 | 11.4 | | | 2.0 | 12129.0 | | |
| 3 (ref) | 87114.0 | 92310.0 | 12.0 | | | 2.0 | 6898.0 | | |
| 4 (ref) | 147743.0 | 178775.0 | 18.7 | | | 3.0 | 7775.0 | | |
| 5 (ref) | 170745.0 | 197209.0 | 16.8 | | | 4.5 | 13670.0 | | |
| 6 (ref) | | | | | | | | | |
| 7 (ref) | 103810.0 | 108394.0 | 10.7 | | | 3.0 | 8532.0 | | |
| 8 (ref) | 200926.0 | 264258.0 | 22.8 | | | 3.0 | 10912.0 | | |
| 9 (ref) | 111382.0 | 116530.0 | 11.2 | | | 2.0 | 10440.0 | | |
| 10 (ref) | 182589.0 | 233712.0 | 23.1 | | | 2.0 | 12522.0 | | |
| 11 (ref) | | | | | | | | | |
| 12 (ref) | 147041.0 | 171197.0 | 16.9 | | | 9.0 | 6350.0 | | |
| AVG(test) | 150510.8 | 185149.0 | 17.4 | | | 8.0 | 7556.7 | 0.790 | 1.116 |
| AVG (ref) | 140626.5 | 165310.1 | 16.1 | | | 3.6 | 9566.9 | | |
| | | | | | | | | | |
| geomn(test) | 145402.2 | 174020.6 | 16.7 | | | 6.3 | 7146.7 | 0.775 | 1.112 |
| geomn(ref) | 136133.6 | 156458.7 | 15.5 | | | 3.2 | 9217.4 | | |
| | | | | | | | | | |
| stddev(test) | 40872 | 67163 | 5.16 | | | 6.44 | 2956.04 | 0.16 | 0.09 |
| stddev(ref) | 37136 | 57016 | 4.66 | | | 2.23 | 2712.70 | | |
| | | | | | | | | | |
| %CV (test) | 27.16% | 36.28% | 0.30 | | | 0.80 | 0.39 | | |
| %CV (ref) | 26.41% | 34.49% | 0.29 | | | 0.62 | 0.28 | | |

## Claims

1. A pharmaceutical composition comprising non-mechanically micronized microparticles of fenofibrate, polyethylene glycol, and polyethylene-polypropylene glycol obtainable by a process which involves sublimation of a sublimable carrier from a solid solution that comprises fenofibrate, the polyethylene glycol, the polyethylene-polypropylene glycol and the sublimable carrier.

2. The pharmaceutical composition of claim 1 wherein the non-mechanically micronized microparticles are deposited on a plurality of pharmaceutical carrier particles.

3. The pharmaceutical composition of claim 1 wherein menthol is the sublimable carrier in the sublimation micronization step.

4. The pharmaceutical composition of any preceding claim wherein the polyethylene glycol is polyethylene glycol 6000

5. The pharmaceutical composition of any preceding claim wherein the polyethylene-polypropylene glycol is poloxamer 407.

6. The pharmaceutical composition of claim 5 wherein the pharmaceutical composition is in the form of a solid oral dosage form comprising about 15% to about 25% by weight fenofibrate, about 7% to about 13% poloxamer 407, and about 7% to about 13% polyethylene glycol 6000.

7. The pharmaceutical composition any preceding claim further comprising a pharmaceutical disintegrant selected from the group consisting of crospovidone, croscarmellose sodium, the bicarbonate or carbonate salts, the organic carboxylic acids, and combinations of any of the foregoing.

8. The pharmaceutical composition of claim 7 wherein the organic carboxylic acids are selected from citric acid, tanic acid, ascorbic acid, benzoic acid, fumaric acid, lactic acid, malic acid, sorbic acid, and tartaric acid, especially citric acid and tartaric acid.

9. The pharmaceutical composition of claim 8 comprising about 12% by weight of either citric acid or tartaric acid, especially about 12% by weight of citric acid.

10. The pharmaceutical composition of claim 7 wherein the bicarbonate or carbonate salts are alkali or alkali earth metal bicarbonates or carbonates such as sodium bicarbonate, sodium carbonate, potassium bicarbonate, potassium carbonate, calcium carbonate magnesium carbonate, especially sodium bicarbonate.

11. The pharmaceutical composition of claim 10 comprising about 12% by weight of sodium bicarbonate.

12. The pharmaceutical composition of any preceding claim further comprising about 15% by weight of microcrystalline cellulose.

13. The pharmaceutical composition of any preceding claim further comprising about 18% by weight of crosspovidone.

14. The pharmaceutical composition of any preceding claim in solid oral dosage form.

15. The solid oral dosage form of claim 14 having a time-dependent *in vitro* fenofibrate release profile such that at least about 51% by weight of the fenofibrate is released in about 10 minutes, at least about 73% by weight of the fenofibrate is released in about 15 minutes, and at least about 85% by weight of the fenofibrate is released in about 30 minutes, and wherein the time-denpendent in vitro release profile is obtained at 37°C using a USP Type-II dissolution tester operating at 50 rpm and filled with 1000 mL of 0.5 wt-% sodium lauryl sulfate in water.

16. The solid oral dosage form of claim 14 having a time-dependent *in vitro* release profile such that about 51% to about 81% by weight of the fenofibrate is released in about 10 minutes, about 73% to about 93% by weight of the fenofibrate is released in about 15 minutes, and about 85% by weight to about all of the fenofibrate is released in about 30 minutes, and wherein the time-dependent *in vitro* release profile is obtained at 37°C using a USP Type-II dissolution tester operating at 50 rpm and filled with 1000 mL of 0.5 wt-% sodium lauryl sulfate in water.

17. A solid oral dosage form comprising a pharmaceutical composition according to any of claims 1 to 13 comprising about 145 mg of sublimation micronized fenofibrate wherein in human *in vivo* pharmacokinetic studies in which the dosage form is administered in the fasted state, the area under the 48-hour AUC curve (AUC₄₈) is about 121367 h*ng/g to about 287539 h*ng/g; the area under the AUC curve extrapolated to infinite time (AUC_{∞}) is about 134750 h*ng/g to about 345390 h*ng/g; and the maximum plasma concentration (Cₘₐₓ) is about 6357 ng/g to about 14627 ng/g.

18. The solid oral dosage form of claim 17 wherein, in human *in vivo* pharmacokinetic studies in which the dosage form is administered in the fasted state, the average AUC₄₈ is about 175335 h*ng/g, the average AUC_{∞} is about 213652 h*ng/g, and the average Cₘₐₓ is about 10570 ng/g.

19. A solid oral dosage form comprising a pharmaceutical composition according to any of claims 1 to 13 comprising about 145 mg of sublimation micronized fenofibrate wherein in human *in vivo* pharmacokinetic studies in which the dosage form is administered in the fed state, the area under the 48-hour AUC curve (AUC₄₈) is about 91601 h*ng/g to about 217512 h*ng/g; and the area under the AUC curve extrapolated to infinite time (AUC_{∞}) is about 97182 h*ng/g to about 308070 h*ng/g.

20. The solid oral dosage form of claim 19 wherein the average AUC₄₈ is about 150511 h*ng/g and the average AUC_{∞} is about 185149 h*ng/g.

21. The solid oral dosage form of any of claims 15 to 2 0 wherein the solid oral dosage form is a compressed tablet.

22. The pharmaceutical composition of any of claims 2 to 16 wherein the combination of fenofibrate, polyethylene glycol, and polyethylene-polypropylene glycol deposited on the plurality of pharmaceutical carrier particles is deposited by sublimation of a sublimable carrier from a solid solution that comprises fenofibrate, the polyethylene glycol, the polyethylene-polypropylene glycol, and the sublimable carrier.

23. The solid oral dosage form of any of claim 1 7to 2 1 wherein the about 145 mg of fenofibrate has been deposited on a plurality of particles of microcrystalline cellulose by sublimation of a sublimable carrier, such as menthol.

24. A process for preparing a pharmaceutical composition comprising sublimation of a sublimable carrier from a solid solution containing fenofibrate, a polyethylene glycol, a polyethylene-polypropylene glycol and a sublimable carrier.

## Patentansprüche

1. Pharmazeutische Zusammensetzung, die nicht mechanisch feinstzerkleinerte Mikropartikel von Fenofibrat, Polyethylenglycol und Polyethylen-Polypropylenglycol umfaßt, die durch ein Verfahren erhältlich sind, welches die Sublimation eines sublimierbaren Trägers aus einer festen Lösung umfaßt, die Fenofibrat, das Polyethylenglycol, das Polyethylen-Polypropylenglycol und den sublimierbaren Träger enthält.

2. Pharmazeutische Zusammensetzung nach Anspruch 1, wobei die nicht mechanisch feinstzerkleinerten Mikropartikel auf einer Mehrzahl von pharmazeutischen Trägerpartikeln abgeschieden sind.

3. Pharmazeutische Zusammensetzung nach Anspruch 1, wobei der sublimierbare Träger in der Stufe der Feinstzerkleinerung mittels Sublimation Menthol ist.

4. Pharmazeutische Zusammensetzung nach einem der vorangegangenen Ansprüche, wobei das Polyethylenglycol Polyethylenglycol 6000 ist.

5. Pharmazeutische Zusammensetzung nach einem der vorangegangenen Ansprüche, wobei das Polyethylen-Polypropylenglycol Poloxamer 407 ist.

6. Pharmazeutische Zusammensetzung nach Anspruch 5, wobei die pharmazeutische Zusammensetzung in Form einer festen oralen Dosierungsform vorliegt, die etwa 15 bis etwa 25 Gewichts-% Fenofibrat, etwa 7% bis etwa 13% Poloxamer 407 und etwa 7% bis etwa 13% Polyethylenglycol 6000 umfaßt.

7. Pharmazeutische Zusammensetzung nach einem der vorangegangenen Ansprüche, welche weiterhin ein pharmazeutisches Sprengmittel umfaßt, ausgewählt aus der Gruppe, bestehend aus Crospovidon, Croscarmellosenatrium, den Bicarbonat- oder Carbonatsalzen, den organischen Carbonsäuren und Kombinationen von beliebigen der vorgenannten.

8. Pharmazeutische Zusammensetzung nach Anspruch 7, wobei die organischen Carbonsäuren ausgewählt sind unter Zitronensäure, Gerbsäure, Ascorbinsäure, Benzoesäure, Fumarsäure, Milchsäure, Äpfelsäure, Sorbinsäure und Weinsäure, insbesondere Zitronensäure und Weinsäure.

9. Pharmazeutische Zusammensetzung nach Anspruch 8, welche etwa 12 Gewichts-% entweder an Zitronensäure oder Weinsäure, insbesondere etwa 12 Gewichts-% Zitronensäure, umfaßt.

10. Pharmazeutische Zusammensetzung nach Anspruch 7, wobei die Bicarbonat- oder Carbonatsalze Alkali- oder Erdalkalimetallbicarbonate oder -carbonate, wie Natriumbicarbonat, Natriumcarbonat, Kaliumbicarbonat, Kaliumcarbonat, Calciumcarbonat, Magnesiumcarbonat, insbesondere Natriumbicarbonat, sind.

11. Pharmazeutische Zusammensetzung nach Anspruch 10, welche etwa 12 Gewichts-% Natriumbicarbonat umfaßt.

12. Pharmazeutische Zusammensetzung nach einem der vorangegangenen Ansprüche, welche weiterhin etwa 15 Gewichts-% mikrokristalline Zellulose umfaßt.

13. Pharmazeutische Zusammensetzung nach einem der vorangegangenen Ansprüche, welche weiterhin etwa 18 Gewichts-% Crospovidon umfaßt.

14. Pharmazeutische Zusammensetzung nach einem der vorangegangenen Ansprüche in fester oraler Dosierungsform.

15. Feste orale Dosierungsform nach Anspruch 14 mit einem zeitabhängigen Fenofibrat-Freisetzungsprofil *in vitro,* so daß wenigstens etwa 51 Gewichts-% des Fenofibrats in etwa 10 Minuten freigesetzt werden, wenigstens etwa 73 Gewichts-% des Fenofibrats in etwa 15 Minuten freigesetzt werden und wenigstens etwa 85 Gewichts-% des Fenofibrats in etwa 30 Minuten freigesetzt werden, und wobei das zeitabhängige Freisetzungsprofil *in vitro* bei 37°C unter Verwendung einer USP Typ II-Auflösungstestvorrichtung erhalten wird, die bei 50 U.p.M. läuft und mit 1000 ml 0,5 gew.-%-igem Natriumlaurylsulfat in Wasser befüllt ist.

16. Feste orale Dosierungsform nach Anspruch 14 mit einem zeitabhängigen Freisetzungsprofil *in vitro,* so daß etwa 51 bis etwa 81 Gewichts-% des Fenofibrats in etwa 10 Minuten freigesetzt werden, etwa 73 bis etwa 93 Gewichts-% des Fenofibrats in etwa 15 Minuten freigesetzt werden und etwa 85 Gewichts-% des Fenofibrats bis etwa das gesamte Fenofibrat in etwa 30 Minuten freigesetzt werden, und wobei das zeitabhängige Freisetzungsprofil *in vitro* bei 37°C unter Verwendung einer USP Typ II-Auflösungstestvorrichtung erhalten wird, die bei 50 U.p.M. läuft und mit 1000 ml 0,5 gew.-%-igem Natriumlaurylsulfat in Wasser befüllt ist.

17. Feste orale Dosierungsform, welche eine pharmazeutische Zusammensetzung nach einem der Ansprüche 1 bis 13 umfaßt, welche etwa 145 mg an mittels Sublimation feinstzerkleinertem Fenofibrat enthält, wobei in humanen pharmakokinetischen Untersuchungen *in vivo*, bei denen die Dosierungsform im nüchternen Zustand verabreicht wird, die Fläche unterhalb der 48-Stunden-AUC-Kurve (AUC₄₈) etwa 121367 h*ng/g bis etwa 287539 h*ng/g beträgt, die Fläche unterhalb der auf einen infiniten Zeitraum extrapolierten AUC-Kurve (AUC_{∞}) etwa 134750 h*ng/g bis etwa 345390 h*ng/g beträgt und die maximale Plasmakonzentration (Cₘₐₓ) etwa 6357 ng/g bis etwa 14627 ng/g beträgt.

18. Feste orale Dosierungsform nach Anspruch 17, wobei in humanen pharmakokinetischen Untersuchungen *in vivo,* bei denen die Dosierungsform im nüchternen Zustand verabreicht wird, die mittlere AUC₄₈ etwa 175335 h*ng/g beträgt, die mittlere AUC_{∞} etwa 213652 h*ng/g beträgt und die mittlere Cₘₐₓ etwa 10570 ng/g beträgt.

19. Feste orale Dosierungsform, welche eine pharmazeutische Zusammensetzung nach einem der Ansprüche 1 bis 13 umfaßt, welche etwa 145 mg an mittels Sublimation feinstzerkleinertem Fenofibrat umfaßt, wobei in humanen pharmakokinetischen Untersuchungen *in vivo,* bei denen die Dosierungsform im ernährten Zustand verabreicht wird, die Fläche unterhalb der 48-Stunden- AUC-Kurve (AUC₄₈) etwa 91601 h*ng/g bis etwa 217512 h*ng/g beträgt und die Fläche unterhalb der auf einen infiniten Zeitraum extrapolierten AUC-Kurve (AUC_{∞}) etwa 97182 h*ng/g bis etwa 308070 h*ng/g beträgt.

20. Feste orale Dosierungsform nach Anspruch 19, wobei die mittlere AUC₄₈ etwa 150511 h*ng/g beträgt und die mittlere AUC_{∞} etwa 185149 h*ng/g beträgt.

21. Feste orale Dosierungsform nach einem der Ansprüche 15 bis 20, wobei die feste orale Dosierungsform eine komprimierte Tablette ist.

22. Pharmazeutische Zusammensetzung nach einem der Ansprüche 2 bis 16, wobei die Kombination aus Fenofibrat, Polyethylenglycol und Polyethylen-Polypropylenglycol, die auf der Mehrzahl von pharmazeutischen Trägerpartikeln abgeschieden ist, mittels Sublimation eines sublimierbaren Trägers aus einer festen Lösung abgeschieden wird, welche Fenofibrat, das Polyethylenglycol, das Polyethylen-Polypropylenglycol und den sublimierbaren Träger umfaßt.

23. Feste orale Dosierungsform nach einem der Ansprüche 17 bis 21, wobei die etwa 145 mg Fenofibrat mittels Sublimation eines sublimierbaren Trägers, wie Menthol, auf einer Mehrzahl von Partikeln aus mikrokristalliner Zellulose abgeschieden wurden.

24. Verfahren zur Herstellung einer pharmazeutischen Zusammensetzung, welches die Sublimation eines sublimierbaren Trägers aus einer festen Lösung, die Fenofibrat, ein Polyethylenglycol, ein Polyethylen-Polypropylenglycol und einen sublimierbaren Träger enthält, umfaßt.

## Revendications

1. Composition pharmaceutique comprenant des microparticules micronisées de manière non mécanique de fénofibrate, de polyéthylèneglycol et de polyéthylène-polypropylèneglycol pouvant être obtenues au moyen d'un procédé qui utilise la sublimation d'un véhicule sublimable à partir d'une solution de matières solides qui comprend le fénofibrate, le polyéthylèneglycol, le polyéthylène-polypropylèneglycol et le véhicule sublimable.

2. Composition pharmaceutique selon la revendication 1, dans laquelle les microparticules micronisées de manière non mécanique sont déposées sur une pluralité de particules d'un véhicule pharmaceutique.

3. Composition pharmaceutique selon la revendication 1, dans laquelle le menthol est le véhicule sublimable de l'étape de micronisation par sublimation.

4. Composition pharmaceutique selon l'une quelconque des revendications précédentes, dans laquelle le polyéthylèneglycol est le polyéthylèneglycol 6000.

5. Composition pharmaceutique selon l'une quelconque des revendications précédentes, dans laquelle le polyéthylène-polypropylèneglycol est le poloxamère 407.

6. Composition pharmaceutique selon la revendication 5, dans laquelle la composition pharmaceutique se présente sous la forme d'une forme galénique orale solide comprenant environ 15% à environ 25% en poids de fénofibrate, environ 7% à environ 13% de poloxamère 407 et environ 7% à environ 13% de polyéthylèneglycol 6000.

7. Composition pharmaceutique selon l'une quelconque des revendications précédentes comprenant en outre un agent de délitement pharmaceutique choisi dans le groupe constitué par la crospovidone, la croscarmellose sodique, les sels d'hydrogénocarbonate ou de carbonate, les acides carboxyliques organiques et les combinaisons de l'un quelconque de ceux-ci.

8. Composition pharmaceutique selon la revendication 7, dans laquelle les acides carboxyliques organiques sont choisi parmi l'acide citrique, l'acide tannique, l'acide ascorbique, l'acide benzoïque, l'acide fumarique, l'acide lactique, l'acide malique, l'acide sorbique et l'acide tartrique, en particulier l'acide citrique et l'acide tartrique.

9. Composition pharmaceutique selon la revendication 8, comprenant environ 12% en poids d'acide citrique ou d'acide tartrique, en particulier environ 12% en poids d'acide citrique.

10. Composition pharmaceutique selon la revendication 7, dans laquelle les sels d'hydrogénocarbonate ou de carbonate sont des carbonates ou des hydrogénocarbonates de métaux alcalino-terreux ou alcalins tels que l'hydrogénocarbonate de sodium, le carbonate de sodium, l'hydrogénocarbonate de potassium, le carbonate de potassium, le carbonate de calcium, le carbonate de magnésium, en particulier l'hydrogénocarbonate de sodium.

11. Composition pharmaceutique selon la revendication 10, comprenant environ 12% en poids d'hydrogénocarbonate de sodium.

12. Composition pharmaceutique selon l'une quelconque des revendications précédentes comprenant en outre environ 15% en poids de cellulose microcristalline.

13. Composition pharmaceutique selon l'une quelconque des revendications précédentes comprenant en outre environ 18% en poids de crospovidone.

14. Composition pharmaceutique selon l'une quelconque des revendications précédentes en forme galénique orale solide.

15. Forme galénique orale solide selon la revendication 14 présentant un profil de libération du fénofibrate *in vitro* dépendant du temps de sorte qu'au moins environ 51% en poids du fénofibrate est libéré en environ 10 minutes, au moins environ 73% en poids du fénofibrate est libéré en environ 15 minutes et au moins environ 85% en poids du fénofibrate est libéré en environ 30 minutes, et dans laquelle le profil de libération *in vitro* dépendant du temps est obtenu à 37°C en utilisant un appareil d'essai de dissolution de type USP II fonctionnant à 50 tr/min et rempli avec 1 000 mL de laurylsulfate de sodium à 0,5% en poids dans de l'eau.

16. Forme galénique orale solide selon la revendication 14 présentant un profil de libération *in vitro* dépendant du temps tel qu'au moins environ 51% à environ 81 % en poids du fénofibrate est libéré en environ 10 minutes, environ 73% à environ 93% en poids du fénofibrate est libéré en environ 15 minutes et environ 85% en poids à environ 100% du fénofibrate est libéré en environ 30 minutes, et dans laquelle le profil de libération *in vitro* dépendant du temps est obtenu à 37°C en utilisant un appareil d'essai de dissolution de type USP II fonctionnant à 50 tr/min et rempli avec 1 000 mL de laurylsulfate de sodium à 0,5% en poids dans de l'eau.

17. Forme galénique orale solide comprenant une composition pharmaceutique selon l'une quelconque des revendications 1 à 13 comprenant environ 145 mg de fénofibrate micronisé par sublimation, dans laquelle, au cours d'études pharmacocinétiques réalisées *in vivo* chez l'Homme dans lesquelles la forme galénique est administrée à l'état de jeûne, l'aire sous la courbe AUC à 48 heures (AUC₄₈) est d'environ 121 367 h*ng/g à environ 287 539 h*ng/g ; l'aire sous la courbe AUC extrapolée à une durée infinie (AUC_{∞}) est d'environ 134 750 h*ng/g à environ 345 390 h*ng/g et la concentration plasmatique maximale (Cₘₐₓ) est d'environ 6 357 ng/g à environ 14 627 ng/g.

18. Forme galénique orale solide selon la revendication 17, dans laquelle, au cours d'études pharmacocinétiques réalisées *in vivo* chez l'Homme dans lesquelles la forme galénique est administrée à l'état de jeûne, l'AUC₄₈ moyenne est d'environ 175 335 h*ng/g, l'AUC_{∞} moyenne est d'environ 213 652 h*ng/g et la Cₘₐₓ moyenne est d'environ 10 570 ng/g.

19. Forme galénique orale solide comprenant une composition pharmaceutique selon l'une quelconque des revendications 1 à 13 comprenant environ 145 mg de fénofibrate micronisé par sublimation, dans laquelle, au cours d'études pharmacocinétiques réalisées *in vivo* chez l'Homme dans lesquelles la forme galénique est administrée à l'état alimenté, l'aire sous la courbe AUC à 48 heures (AUC₄₈) est d'environ 91 601 h*ng/g à environ 217 512 h*ng/g ; et l'aire sous la courbe AUC extrapolée à une durée infinie (AUC_{∞}) est d'environ 97 182 h*ng/g à environ 308 070 h*ng/g.

20. Forme galénique orale solide selon la revendication 19, dans laquelle l'AUC₄₈ moyenne est d'environ 150 511 h*ng/g et l'AUC_{∞} moyenne est d'environ 185 149 h*ng/g.

21. Forme galénique orale solide selon l'une quelconque des revendications 15 à 20, dans laquelle la forme galénique orale solide est un comprimé.

22. Composition pharmaceutique selon l'une quelconque des revendications 2 à 16, dans laquelle la combinaison de fénofibrate, de polyéthylèneglycol et de polyéthylène-polypropylèneglycol déposée sur la pluralité de particules d'un véhicule pharmaceutique est déposée par sublimation d'un véhicule sublimable à partir d'une solution de matières solides qui comprend le fénofibrate, le polyéthylèneglycol, le polyéthylène-polypropylèneglycol et le véhicule sublimable.

23. Forme galénique orale solide selon l'une quelconque des revendications 17 à 21, dans laquelle les environ 145 mg de fénofibrate ont été déposés sur une pluralité de particules de cellulose microcristalline par sublimation d'un véhicule sublimable, tel que le menthol.

24. Procédé de préparation d'une composition pharmaceutique comprenant la sublimation d'un véhicule sublimable à partir d'une solution de matières solides contenant du fénofibrate, un polyéthylèneglycol, un polyéthylène-polypropylèneglycol et un véhicule sublimable.
